# EUROPÄISCHE PATENTANMELDUNG

(11) **EP 2 168 491 A1**
(43) Veröffentlichungstag der Anmeldung: **31.03.2010**
(21) Anmeldenummer: 09154863.6
(22) Anmeldetag: 11.03.2009
(51) Int. Cl.: A61B 6/03, A61B 10/00, G01N 23/04, A61B 6/00, A61B 6/02, A61B 6/04, G01N 1/00, A61B 10/02, H01J 37/16, B01L 3/00

(54) **Brustfixierung mit Probencontainer für ein Untersuchungsgerät der weiblichen Brust**

(30) Priorität: 29.09.2008 DE 102008042430
(71) Anmelder: MIR Medical Imaging Research Holding GmbH, 91096 Möhrendorf (DE)
(72) Erfinder: Kalender, Willi, 91096, Möhrendorf (DE); Schilling, Harry, 85072, Eichstätt (DE)
(74) Vertreter: Lohr, Georg

(57) **Zusammenfassung**

Ein Röntgengerät basierend auf einem Spiral-CT Scanner zur Untersuchung einer weiblichen Brust hat eine drehbare Gantry (10), die gleichzeitig senkrecht zur Drehachse verschiebbar ist. Ein zylinderförmiger Abbildungsbereich wird in Form einer Spirale abgetastet. Die Patientin wird der Art gelagert, dass die zu untersuchende Brust in den Abbildungsbereich hineinragt. Weiterhin befindet sich in dem Abbildungsbereich ein Probencontainer (83). In dem Probencontainer kann eine Gewebeprobe (32) oder auch Referenzmaterial (85) aufgenommen werden.

## Beschreibung

### Technisches Gebiet

Die Erfindung betrifft ein Röntgengerät zur Abbildung einer weiblichen Brust (Mammografie).

### Stand der Technik

Zur Untersuchung der weiblichen Brust sind verschiedene Geräte, wie Röntgengeräte oder auch CT-Scanner bekannt. Ein solcher CT-Scanner ist beispielsweise in der US 2006/0094950 A1 offenbart. Unter einer Liege, auf der eine zu untersuchende Patientin liegt, befindet sich eine Röntgenvorrichtung mit einer rotierenden Gantry, welche eine Röntgenröhre und einen Detektor aufweist. Die zu untersuchende Brust der Patientin ragt durch eine Öffnung in der Liege in den Strahlengang der Röntgenvorrichtung.

### Darstellung der Erfindung

Der Erfindung liegt die Aufgabe zugrunde, ein Röntgengerät sowie ein Verfahren zum Betrieb eines Röntgengerätes zur Untersuchung der Brust einer Patientin derart auszugestalten, dass eine höhere Genauigkeit und Auflösung sowie eine bessere diagnostische Aussage erreicht werden kann. Ein weiterer Aspekt der Erfindung ist die entsprechende Ausgestaltung einer Fixierung einer weiblichen Brust.

Diese Aufgabe wird durch die Gegenstände der unabhängigen Ansprüche gelöst. Vorteilhafte Ausgestaltungen der Erfindung sind in den Unteransprüchen angegeben.

Ein erfindungsgemäßes Röntgengerät zur Untersuchung der Brust 31 einer Patientin 30 umfasst eine Spiral-CT (Computertomographen) Gantry 10. Diese Gantry ist um eine Rotationsachse 12 drehbar und gleichzeitig beziehungsweise zumindest korreliert mit der Drehung senkrecht zur Drehachse verschiebbar. Bevorzugt erfolgt die Verschiebung gleichzeitig mit der Drehbewegung, so dass sich eine spiralförmige Bahn um einen zylinderförmigen Abbildungsbereich ergibt. Das Röntgengerät hat weiterhin eine Auflagefläche 20, an der die Patientin 30 derart angeordnet werden kann, dass die zu untersuchende Brust 31 in den Abbildungsbereich der Gantry hineinragt. Weiterhin weist das erfindungsgemäße Röntgengerät noch einen Probencontainer 83 auf, welcher ebenfalls im Abbildungsbereich der Gantry angeordnet ist. So kann in einem ersten Abbildungsschritt die Brust der Patientin erfasst werden. In einem zweiten Abbildungsschritt kann die Gewebeprobe beziehungsweise das Referenzmaterial in dem Probencontainer untersucht werden. Für diesen zweiten Abbildungsschritt wird das Röntgengerät bevorzugt in einen hochauflösenden Betriebsmodus versetzt. Beim Scannen (=abbilden) der Brust muss innerhalb einer typischen Zeitspanne von 10 Sekunden das gesamte Brustvolumen erfasst werden, da die Patientin während der gesamten Zeitspanne nicht atmen darf. Im Gegensatz hierzu steht für das wesentlich kleinere Volumen der der Gewebeprobe beziehungsweise des Referenzmaterials eine wesentlich längere Scandauer zur Verfügung. Somit kann mit einem deutlich kleineren Fokuspunkt der Röntgenstrahlung gearbeitet werden. Weiterhin müssen nicht die gesamten Gewebeschichten der Brust durchstrahlt werden. Zudem ist aufgrund der geringen Größe der Gewebeprobe beziehungsweise der Referenzmaterialien im Normalfall kein Spiral-Scan notwendig. Die Gantry kann also auf einer Kreisbahn bewegt werden oder sogar in einer festen Position bleiben. Insgesamt ergibt sich dadurch eine wesentlich höhere Auflösung als bei einer Abbildung der Brust. Man kann hier exakte diagnostische Informationen über die Art und Morphologie des Gewebes der Gewebeprobe beziehungsweise eines darin enthaltenen Tumors gewinnen. Weiterhin lassen sich Vergleiche mit hoher Genauigkeit zwischen dem Brustgewebe und den Inhalt des Probencontainers durchführen. Toleranzen, die durch zeitlich versetzte Aufnahmen von Brust und Probe in herkömmlichen Geräten entstehen, treten hier nicht auf. Weiterhin kann mit geringem Zeitversatz mit der Brust auch Referenzmaterial aufgenommen werden. Dies erlaubt eine besonders präzise Kalibrierung des Röntgengerätes. Dadurch lassen sich hochwertigere Bilder mit einer besseren diagnostischen Aussage erzeugen.

Grundsätzlich kann mit einem erfindungsgemäßen Röntgengerät auch eine dreidimensionale Untersuchung einer Gewebeprobe ohne die Patientin erfolgen. Es ist hier kein separates Gerät zur Untersuchung der Gewebeprobe erforderlich.

Mit einem erfindungsgemäßen Röntgengerät kann auch eine Gewebeprobe, die gerade von einer an einer Auflagefläche 20 positionierten Patientin entnommen wurde, röntgentechnisch untersucht werden. Hierbei ist es nicht notwendig, dass die Patientin die Auflagefläche 20 verlässt.

Vorteilhafterweise ist die Gantry 10 derart konfiguriert, dass ein Probenadapter 82 zur Aufnahme des Probencontainers 83 in die Gantry eingesetzt werden kann. Dadurch ist keine separate Halterung für den Probencontainer notwendig. Vielmehr wird der Probenadapter vor der Untersuchung in die Gantry selbst oder eine Halterung in der Gantry eingesetzt.

Besonders vorteilhaft ist es, wenn eine Biopsieeinrichtung vorhanden ist. So kann mittels einer Biopsienadel Gewebe aus der Brust entnommen werden. Dieses wird dann in die Probenaufnahme bzw. in den Probencontainer transportiert. Dort kann es dann durch die Gantry abgebildet werden.

Besonders vorteilhaft ist es, wenn eine Unterdruck-Biopsieeinrichtung vorhanden ist. So kann mittels einer Unterdruck-Biopsienadel Gewebe aus der Brust entnommen werden. Dieses wird dann mittels Unterdruck in die Probenaufnahme bzw. in den Probencontainer transportiert. Dort kann es dann durch die Gantry abgebildet werden.

Besonders vorteilhaft ist es, wenn der Probenadapter 82 eine Strahlenabschirmung 84 aufweist, so dass bei einer Abbildung des Inhalts des Probencontainers die Strahlenbelastung für die knapp darüber liegende Patientin minimal ist.

In einer weiteren vorteilhaften Ausgestaltung der Erfindung weist wahlweise der Probenadapter 82 und/oder die Probenaufnahme 83 wenigstens ein automatisches Identifikationssystem, welches einen RFID-Transponder umfassen könnte, wenigstens einen RFID-Transponder 90 und/oder wenigstens einen Barcode 94 und/oder eine beliebige andere Kennzeichnung auf. Damit ist dann die Probe eindeutig identifizierbar.

Ein weiterer Aspekt Erfindung betrifft einen Probenadapter 82 zum Einsatz in einem zuvor geschilderten Röntgengerät. Der Probenadapter weist Raum für eine Probenaufnahme 83 auf. Weiterhin ist es vorteilhaft, wenn der Probenadapter 82 und/oder die Probenaufnahme 83 wenigstens ein automatisches Identifikationssystem, welches einen RFID-Transponder umfassen könnte, wenigstens einen RFID-Transponder 90 und/oder wenigstens einen Barcode 94 und/oder eine beliebige andere Kennzeichnung aufweisen.

Ein weiterer Aspekt der Erfindung umfasst eine Vorrichtung zur Fixierung einer weiblichen Brust 31 einer Patientin 30 in einem Röntgengerät. Die Vorrichtung zur Fixierung der Brust weist eine Probenaufnahme 83 und/oder einen Probenadapter 82 auf, in die eine Gewebeprobe 32 und/oder ein Referenzmaterial 85 eingebracht werden kann. Diese Gewebeprobe und oder das Referenzmaterial können dann gleichzeitig in einem Scanvorgang zusammen mit der Brust der Patientin gescannt und abgebildet werden. Es ist vorteilhaft, wenn die Vorrichtung zur Fixierung, und/oder der Probenadapter 82 und/oder die Probenaufnahme 83 wenigstens ein automatisches Identifikationssystem, welches einen RFID-Transponder umfassen könnte, wenigstens einen RFID-Transponder 90 und/oder wenigstens einen Barcode 94 und/oder eine beliebige andere Kennzeichnung aufweisen.

Ein erfindungsgemäßes Röntgenverfahren wird mit einem Spiral-CT Scanner zur Untersuchung einer weiblichen Brust 31 durchgeführt. Dieser Scanner hat eine Gantry 10, welche um die Brust drehbar ist und gleichzeitig beziehungsweise korreliert mit der Drehung im Abstand zur Brust verschiebbar ist. Die Abtastung der Brust erfolgt typischerweise in einer spiralförmigen Kurvenform.

Das erfindungsgemäße Verfahren umfasst die folgenden Schritte:
1. Positionieren der Patientin 30 an einer Auflagefläche 20 derart, dass die zu untersuchende Brust 31 in den Abbildungsbereich ragt.
2. Röntgenabbildung der Brust durch die Gantry 10.
3. Entnahme einer Gewebeprobe aus der Brust an einer durch die Röntgenabbildung lokalisierten Stelle.
4. Röntgenabbildung der Gewebeprobe durch die Gantry, vorzugsweise in einer Betriebsart mit höherer Auflösung.

Die wichtigsten Schritte des Verfahrens sind die Schritte 2 und 4. Die Schritte 1 und 3 können gegebenenfalls modifiziert oder weggelassen werden. Das erfindungsgemäße Verfahren ist unabhängig von der Lage der Patientin beziehungsweise des Röntgengerätes. Somit ist jedes anwendbar bei Röntgengeräten, bei denen die Patientin auf einer horizontalen Liege liegt. Ebenso ist es anwendbar bei vertikal angeordneten Röntgengeräten, bei denen die Patientin vor dem Röntgengerät steht. Selbstverständlich ist es auch bei beliebigen geneigten Röntgengeräten anwendbar.

### Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben.
Figur 1 zeigt eine Erfindungsgemäße Vorrichtung.
Figur 2 zeigt einen Schnitt durch einen Probenadapter.
Figur 3 zeigt eine Probenaufnahme.
Figur 4 zeigt eine Vorrichtung mit Vakuumbiopsieeinrichtung
Figur 5 zeigt eine Erfindungsgemäße Vorrichtung mit Brustfixierung.

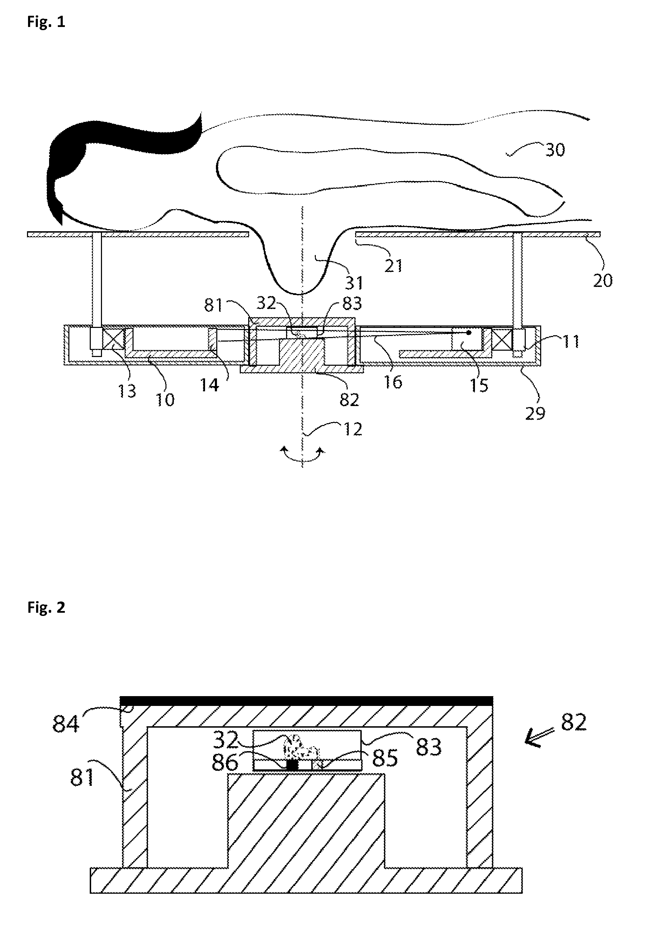

In Figur 1 ist ein seitlicher Schnitt eines erfindungsgemäßen Röntgengerätes dargestellt. Die Patientin 30 ist auf der Patientenliege 20 gelagert. Unterhalb der Patientin befindet sich eine Spiral-Computertomographen Gantry 10. Die Brust 31 der Patientin hängt durch den Brustausschnitt 21 in den Aufnahmebereich dieser Gantry. Die Gantry 10 hat innerhalb des Gantrygehäuses 29 eine Röntgenröhre 15, welche den Strahlenfächer 16 erzeugt. Die Strahlung dieses Strahlenfächers durchdringt die Brust 31 und wird von dem Detektor 14 aufgefangen. Um die gesamte Brust abzubilden, lässt die Gantry sich durch das Gantrydrehlager 13 um die Rotationsachse 12 drehen. Gleichzeitig mit der Drehung wird über den Gantryhubantrieb 11 eine Verschiebung senkrecht zur Drehachse, hier in vertikaler Richtung, durchgeführt, so dass die Brust spiralförmig abgetastet wird. Erfindungsgemäß befindet sich im Erfassungsbereich der Gantry neben der Brust 31 noch ein Probencontainer 83 mit einer Gewebeprobe 32. Anstelle einer Gewebeprobe kann hier auch eine Materialprobe oder ein beliebiges Referenzmaterial eingebracht sein. Der Probenadapter 82 hat eine Probenaufnahme 83, auf der die Gewebeprobe 32 angeordnet ist. Über der Gewebeprobe befindet sich eine Abdeckung 81. In dieser Figur ist eine Position der Gantry 10 dargestellt, in der die Gewebeprobe 32 untersucht werden kann. Im gleichen Scanvorgang kann auch die Brust 31 der Patientin 30 untersucht werden. Hierzu muss nur die Gantry mittels des Gantryhubantriebs nach oben, in Richtung zur Brust verfahren werden. Somit können mit einem einzigen Scan der Gantry die Brust 31 und die Gewebeprobe 32 erfasst werden. Alternativ kann der Probencontainer 83 auch mittels eines Probenadapters 82 in die Gantry 10 eingesetzt werden. Vorzugsweise sind noch Mittel zur Fixierung des Probenadapters in der Gantry, wie beispielsweise Schrauben, Magnete oder auch ein Bajonettverschluss vorgesehen. In diesem Falle erfolgt ein separater Scanvorgang für den Inhalt des Probencontainers.

In Figur 2 ist ein detaillierter Schnitt durch den Probenadapter 82 dargestellt. Hier ist oberhalb der Abdeckung 81 noch eine Strahlenabschirmung 84 angebracht. Diese verhindert eine zusätzliche Strahlenbelastung der Patientin während der Bestrahlung der Proben. In der Probenaufnahme 83 sind neben einer Gewebeprobe 32 noch ein erstes Referenzmaterial 85 sowie ein zweites Referenzmaterial 86 angeordnet.

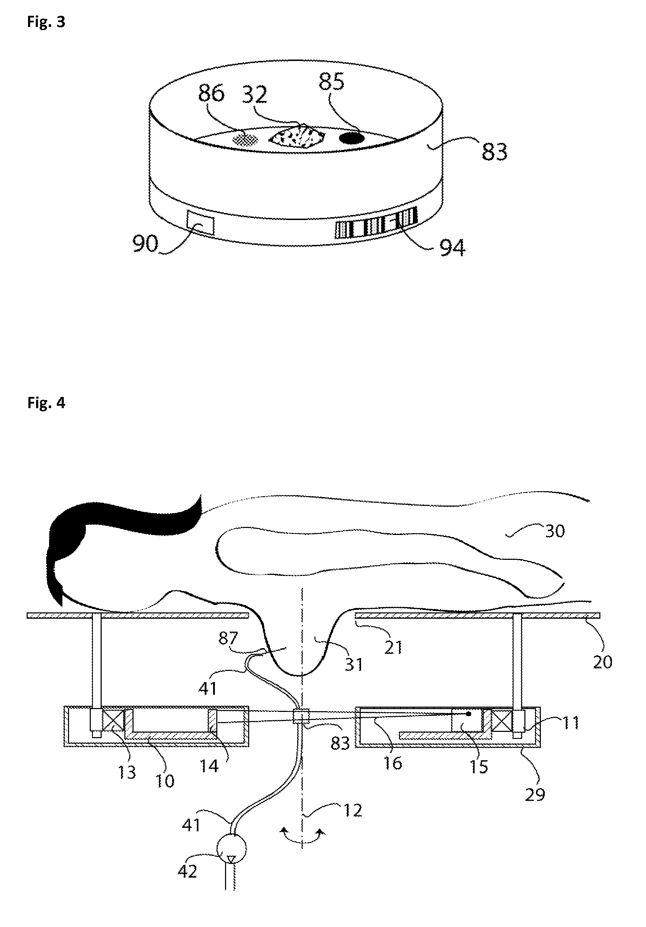

Figur 3 zeigt eine Probenaufnahme 83. Diese Probenaufnahme 83 ist im Wesentlichen ein Behälter, in dem hier beispielhaft eine Gewebeprobe 32, ein erstes Referenzmaterial 85 und ein zweites Referenzmaterial 86 enthalten sind. Zur eindeutigen Identifizierung beziehungsweise Zuordnung ist an der Probenaufnahme noch ein RFID Transponder 90 sowie ein Barcode 94 (Strichcode) angebracht.

Figur 4 zeigt eine weitere Ausgestaltung eines erfindungsgemäßen Röntgengerätes mit einer Vakuum-Biopsieeinrichtung. Mittels der Vakuum-Biopsienadel 87 können Gewebeproben aus der Brust entnommen werden. Diese Vakuum-Biopsienadel 87 ist mit der Probenaufnahme 83 und über einen Schlauch 41 mit der Vakuumpumpe 42 verbunden. Die durch die Vakuum-Biopsienadel 87 zuerst ausgeschnittenen Gewebeproben werden dann durch das Vakuum in die Probenaufnahme 83 befördert. Dort können sie mittels der Gantry 10 untersucht werden.

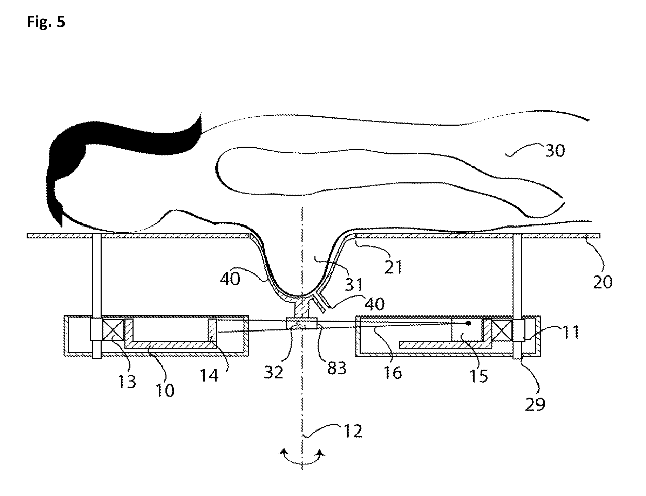

Figur 5 zeigt eine weitere Ausgestaltung eines erfindungsgemäßen Röntgengerätes. Hierin ist eine Vorrichtung zur Brustfixierung in Form eines Bechers 40 in der Auflagefläche 20 befestigt. Der Becher 40 kann auch noch einen Anschluss für eine Vakuumpumpe aufweisen. Zudem ist an dem Becher, vorzugsweise in der Rotationsachse 12 der Gantry 10 ein Probencontainer 83 vorgesehen.

### Bezugszeichenliste

- 10: Gantry
- 11: Gantryhubantrieb
- 12: Rotationsachse
- 13: Gantrydrehlager
- 14: Detektor
- 15: Röntgenröhre
- 16: Strahlenfächer
- 20: Auflagefläche
- 21: Brustausschnitt
- 29: Gantrygehäuse
- 30: Patientin
- 31: Brust
- 32: Gewebeprobe
- 40: Becher
- 41: Schlauch
- 42: Vakuumpumpe
- 81: Abdeckung
- 82: Probenadapter
- 83: Probenaufnahme
- 84: Strahlenabschirmung
- 85: Erstes Referenzmaterial
- 86: Zweites Referenzmaterial
- 87: Vakuum-Biopsienadel
- 90: RFID Transponder
- 94: Barcode

## Patentansprüche

1. Röntgengerät zur Abbildung einer Brust (31) einer Patientin (30), umfassend
- eine Gantry (10), welche um eine Rotationsachse (12) drehbar und gleichzeitig senkrecht zur Rotationsachse verschiebbar ist und somit einen zylinderförmigen Abbildungsbereich abdeckt,
- eine Auflagefläche (20), an der die Patientin (30) derart angeordnet werden kann, dass die zu untersuchende Brust (31) in den Abbildungsbereich der Gantry (10) hineinragt,
**dadurch gekennzeichnet, dass**
ein Probencontainer (83) im Abbildungsbereich der Gantry (10) angeordnet ist, in dem wenigstens eine Gewebeprobe (32) und/oder wenigstens ein Referenzmaterial (85) aufgenommen werden können, und der Bereich der Verschiebung der Gantry so dimensioniert ist, dass die Brust (31) und /oder der Inhalt des Probencontainers (83) abgebildet werden können.

2. Röntgengerät nach Anspruch 1,
**dadurch gekennzeichnet, dass**
die Gantry eine Betriebsart mit kleinerem Fokuspunkt als bei der Abbildung der Brust zur Abbildung von Gewebeprobe (32) und/oder wenigstens Referenzmaterial (85) aufweist.

3. Röntgengerät nach Anspruch 1 oder 2,
**dadurch gekennzeichnet, dass**
ein Probenadapter (82) zur Aufnahme des Probencontainers (83) in die Gantry (10) einsetzbar ist.

4. Röntgengerät nach Anspruch 3,
**dadurch gekennzeichnet, dass**
der Probenadapter (82) eine Strahlenabschirmung (84) umfasst, welcher die Strahlenbelastung der Patientin während des Scannens des Inhalts des Probencontainers reduziert.

5. Röntgengerät nach Anspruch 3,
**dadurch gekennzeichnet, dass**
der Probenadapter (82) wenigstens ein automatisches Identifikationssystem und/oder einen RFID-Transponder (90) und/oder einen Barcode (94) und/oder eine Kennzeichnung aufweist, welche eine Identifizierung des Probenadapters und/oder seines Inhaltes ermöglichen.

6. Röntgengerät nach Anspruch 1 oder 2,
**dadurch gekennzeichnet, dass**
ein Becher (40) zur Brustfixierung vorgesehen ist, an welchem der Probencontainer (83) befestigt ist.

7. Röntgengerät nach Anspruch 1,
**dadurch gekennzeichnet, dass**
der Probencontainer (83) wenigstens ein automatisches Identifikationssystem und/oder einen RFID-Transponder (90) und/oder einen Barcode (94) und/oder eine Kennzeichnung aufweist, welche eine Identifizierung des Probencontainers und/oder seines Inhaltes ermöglichen.

8. Röntgengerät nach einem der Ansprüche 1 bis 5,
**dadurch gekennzeichnet, dass**
eine Biopsieeinrichtung mit einer Biopsienadel vorgesehen ist, wobei eine durch die Biopsienadel aus der Brust entnommene Gewebeprobe in den Probencontainer (83) befördert wird.

9. Röntgengerät nach Anspruch 8,
**dadurch gekennzeichnet, dass**
eine Vakuumbiopsieeinrichtung mit einer an eine Vakuumpumpe angeschlossenen Biopsienadel vorgesehen ist, wobei eine durch die Biopsienadel aus der Brust entnommene Gewebeprobe mit Hilfe des Vakuums in den Probencontainer (83) befördert wird.

10. Probenadapter für ein Röntgengerät nach Anspruch 1,
**dadurch gekennzeichnet, dass**
der Probenadapter (82) Raum für einen Probencontainer (83) und eine Strahlenabschirmung (84) aufweist.

11. Probenadapter nach Anspruch 10,
**dadurch gekennzeichnet, dass**
der Probenadapter (82) und/oder eine in diesem angeordneter Probencontainer (83) ein automatisches Identifikationssystem und/oder wenigstens einen RFID-Transponder (90) und/oder einen Barcode (94) und/oder eine Kennzeichnung aufweist, welche eine Identifizierung des Probenadapters und/oder seines Inhaltes ermöglichen.

12. Vorrichtung zur Fixierung der weiblichen Brust in einem als Spiral-CT ausgebildeten Röntgengerät zur Abbildung der weiblichen Brust umfassend einen Becher (40) zur Aufnahme der Brust (31) und einen Probencontainer (83) in dem wenigstens eine Gewebeprobe (32) und/oder wenigstens ein Referenzmaterial (85) aufgenommen werden können.

13. Vorrichtung nach Anspruch 12,
**dadurch gekennzeichnet, dass**
die Vorrichtung zur Fixierung der weiblichen Brust und/oder der Probenadapter (82) und/oder ein in diesem angeordneter Probencontainer (83) wenigstens einen RFID-Transponder (90) und/oder einen Barcode (94) und/oder eine Kennzeichnung aufweist, welche eine Identifizierung der Vorrichtung zur Fixierung der weiblichen Brust und/oder des Probenadapters und/oder seines Inhaltes ermöglichen.

14. Röntgenverfahren mit einem Spiral-CT Scanner mit einer Gantry (10) zur Untersuchung einer weiblichen Brust (31) einer Patientin (30), **gekennzeichnet durch** die Schritte:
- Positionieren der Patientin (30) an einer Auflagefläche (20) derart, dass die zu untersuchende Brust (31) in den Abbildungsbereich der Gantry (10) ragt.
- Röntgenabbildung der Brust **durch** die Gantry (10).
- Entnahme einer Gewebeprobe aus der Brust an einer **durch** die Röntgenabbildung lokalisierten Stelle.
- Röntgenabbildung der Gewebeprobe **durch** die Gantry, vorzugsweise in einer Betriebsart mit höherer Auflösung.

15. Röntgenverfahren nach Anspruch 13,
**gekennzeichnet durch** den Einsatz eines Röntgengerätes nach Anspruch 1.
